# EUROPEAN PATENT SPECIFICATION

(11) **EP 4 188 503 B1**
(45) Date of publication and mention of the grant of the patent: **21.10.2026**
(21) Application number: 21849814.5
(22) Date of filing: 27.07.2021
(51) Int. Cl.: A61M 25/00, A61M 25/01, A61B 17/12

(54) **EMBOLIZATION MICROCATHETER FOR DELIVERY OF LARGE BEADS**
EMBOLISATIONSMIKROKATHETER ZUR FREISETZUNG GROSSER KÜGELCHEN
MICROCATHÉTER D'EMBOLISATION POUR L'ADMINISTRATION DE GROSSES BILLES

(30) Priority: 30.07.2020 IL 27641620
(43) Date of publication of application: 07.06.2023
(73) Proprietor: Accurate Medical Therapeutics Ltd., 7608802 Rehovot (IL)
(72) Inventor: DAGAN, Tom, 8496500 Omer (IL); ZIPORY, Yuval, 7175472 Modiin (IL); HARBATER, Osnat, 4358142 Raanana (IL); MILLER, Eran, 7680300 Moshav Beit Elazari (IL)
(74) Representative: AWA Denmark A/S
(86) International application number: PCT/IL2021/050904
(87) International publication number: WO 2022/024117

(56) References cited:
- WO-A1-2019/087191
- WO-A1-2019/087191
- CN-A- 101 631 584
- US-A1- 2015 005 801
- US-A1- 2018 250 498
- US-A1- 2019 217 052
- US-A1- 2020 230 355
- US-A1- 2020 230 355

## Description

### TECHNICAL FIELD

The present disclosure generally relates to the field of microcatheters for embolization, specifically to large diameter embolization catheters suitable for delivery through standard delivery catheters.

### BACKGROUND

Transarterial embolization therapy, tumor embolization, or transcatheter arterial embolization (TAE), involves administration of embolization material (which may include chemotherapeutics or/and radiotherapeutics) directly to a tumor (for example, liver tumors), via a microcatheter.

Embolization of tumors is typically performed utilizing microcatheters due to the requirement for selectively affecting the tumor while preventing, as much as possible, damage to healthy tissue. A major problem associated with embolization is "non-target embolization," where the embolic material travels to blood vessels, other than those directly feeding the target tumor or tumor region, thus damaging healthy tissues, resulting in unpleasant and even hazardous outcomes. Possible scenarios include gastric ulcers caused by liver embolization, as well as cases where embolic material refluxes alongside the microcatheter reaching the wall of the stomach, possibly causing ischemia and ulceration. An additional phenomenon, which is abundant especially in advanced stage liver cancer, is non-target embolization through arterioportal shunt.

US 2018/0250498 A1 discloses a catheter with an elongated body comprising proximal and distal portions. The distal portion of the elongated body comprises an inner liner that includes a proximal liner section and a distal liner section that include different materials, and an outer jacket positioned over the inner liner. The distal liner section has a first hardness and the proximal liner section has a second hardness, where the first hardness is less than the second hardness.

A microcatheter is usually passed via a larger-lumen catheter, which is placed within the proximal part of the vessel, such as the celiac or hepatic artery, and the microcatheter is then advanced therethrough towards the tumor until reaching a target location. In some scenarios, it is advantageous to use a diagnostic catheter as the delivery medium for the microcatheter. This procedure withholds the need of replacing one catheter with another, thus saving substantial time.

Another reason that microcatheters are routinely used in embolization procedures is the size of the feeding vessels, which carry blood directly to the organ or tumor. In order to reach as close as possible to the tumor, the embolization catheter is advanced into smaller and sometimes tortuous vessels. Accessibility to these vessels is difficult, if not precluded, with a larger and often stiffer catheter. Moreover, blood vessels in the body tend to go into spasm when manipulated, causing an ineffective embolic material delivery, so flexible micro-sized catheters are an absolute necessity.

A major drawback of trans-catheter embolization is that the embolization material, which is typically invisible, can be refluxed and reach non-target tissue and cause damage to them. In addition, reflux of embolization material may negatively affect the delivery of the embolization material to the target tissue, and thus impair treatment effectiveness and its clinical outcome.

Microcatheters with filter sections for delivery of embolization beads, while preventing backflow of the beads, have been disclosed by the inventors of the present applications. However, such catheters proved unsuitable for delivery of large embolization beads due to the beads clogging the microcatheter, while a simple enlarging of the outer diameter of the microcatheter makes them unsuitable for delivery through standard delivery catheters, such as 5.0 French delivery catheters.

### SUMMARY OF THE INVENTION

The invention is defined in the independent claim 1. Advantageous embodiments are set out in the dependent claims. The present disclosure relates to embolization microcatheters which are suitable for delivery through 5.0 French delivery catheters, while also facilitating delivery of large embolization beads, i.e. beads with a particle size of 900 microns or more.

This is advantageously achieved by the unique structure of the wall of the embolization microcatheters.

The wall is made of polymeric material(s) formed around a braid which provide structural integrity to the microcatheter (especially along the filter section thereof) as well as trackability. The wall also includes an inner liner, coating the inner surface of the wall, and configured to reduce the friction between the wall and the beads flowing therethrough. Yet, the thickness of the wall is maintained below about 120 microns (at least along the portion thereof passing through the delivery microcatheter). The main obstacle to maintaining the thinness of the wall is the radiopaque marker located in proximity to the distal end opening of the microcatheter. It was surprisingly found by the inventors of the hereindisclosed microcatheters that the desired wall thinness may be accomplished and maintained, by having the inner liner extending only up until the radiopaque marker so as to avoid bulking, and that absence of a liner along the part of the microcatheter extending from the radiopaque marker to the distal end opening, has minimal to no influence on the delivery of the beads through the distal end opening.

The hereindisclosed embolization microcatheters may further include a filter section with a plurality of openings configured for outflow of fluids while preventing outflow of the beads, thus ensuring concentrated delivery of the embolization beads with minimal backflow.

Advantageously, the size, shape and distribution of the openings enables smooth delivery of the beads, despite their large size. This is accomplished by including two or more filter segments, each filter segment comprising a plurality of side openings having the proximal most of the circumferential sections distributed circumferentially around the microcatheter, wherein the proximal most of the circumferential sections includes fewer side openings than sections distal thereto. As a result, the outflow of fluid is relatively low at the proximal end of the filter, thus reducing the chance of clogging, and increases toward the distal end opening at which a low outflow rate is desired in order to prevent backflow. In addition, the shape and size of the openings change between the proximal end and the distal end of the filter in that the distal most part of the filter includes small, preferably square-shaped openings configured to prevent the beads to get stuck and/or sucked in as the amount of suspension fluid is reduced.

Some aspects of the present disclosure provide an embolization microcatheter comprising: an elongated tubular member forming a lumen, the elongated tubular member terminating with a distal end opening, wherein a wall of the elongated tubular member comprises a braid, a polymer formed around the braid and an inner liner coating an inner surface of the wall; wherein the wall of the part of the elongated tubular member extending between a proximal radiopaque marker and the distal end opening has a thickness of less than or equal to 130 microns; and a distal radiopaque marker located in proximity to the distal end opening; wherein the part of elongated tubular member extending between the proximal end of the radiopaque marker and the distal end opening is devoid of the liner.

According to some embodiments, the outer diameter of the elongated tubular member is less than or equal to 1.5 mm and an inner diameter of the elongated tubular member is 700 microns or more.

According to some embodiments, the thickness of the distal end of the wall is less than or equal to 120 microns.

According to some embodiments, the embolization microcatheter includes a filter formed in the wall of the elongated tubular member, the filter comprising two or more filter segments, each filter segment comprising a plurality of side openings penetrating the wall, the plurality of side openings distributed circumferentially around the elongated tubular member.

According to some embodiments, the proximal most of the filter segments comprises fewer side openings than rings distal thereto.

According to some embodiments, the distal most of the filter segment is positioned 2-4 mm proximally to the distal end opening.

According to some embodiments, the side openings of the distal most filter segment are essentially square shaped. According to some embodiments, the essentially square shaped side openings have a dimension of 50x50 microns. According to some embodiments, the essentially square shaped side openings have a cross-sectional dimension of 80x50 microns.

According to some embodiments, the side openings of the remainder of the at least two filter segments are in a form of axial slits.

According to some embodiments, the side openings of the remainder of the at least two filter segments have a width of 20 microns and wherein the length of the side openings of the remainder of the at least two filter segments varies between the side openings.

According to some embodiments, the side openings of the remainder of the at least two filter segments have a length of at least 200 microns.

According to some embodiments, at least some of the side openings of the remainder of the at least two filter segments have dimension of 2700x20 microns.

According to some embodiments, at least some of the side openings of the remainder of the at least two filter segments have a dimension of 1350x20 microns. According to some embodiments, at least some of the side openings of the remainder of the at least two filter segments have a dimension of 300x20 microns.

According to some embodiments, the braid is made of tungsten.

According to some embodiments, the inner liner comprises Polytetrafluoroethylene (PTFE).

According to some embodiments, the radiopaque marker comprises a metal marker band. According to some embodiments, the radiopaque marker is located 0.5-2 mm proximally to the distal end opening.

According to some embodiments, the embolization microcatheter is suitable for delivery of embolization beads having a particle size of about 900 microns.

According to some embodiments, the embolization microcatheter has a length of at least 1 m.

According to some embodiments, there is provided a method for delivering embolization beads, the method comprising: delivering the hereindisclosed embolization microcatheter to a target location via a delivery catheter; and injecting beads through the microcatheter.

According to some embodiments, the beads have a particle size of at least 500 microns. According to some embodiments, the beads have a particle size of at least 900 microns.

According to some embodiments, the delivery catheter is a 5.0 Fr delivery catheter.

Certain embodiments of the present disclosure may include some, all, or none of the above characteristics. One or more technical advantages may be readily apparent to those skilled in the art from the figures, descriptions and claims included herein. Moreover, while specific characteristics have been enumerated above, various embodiments may include all, some or none of the enumerated characteristics.

In addition to the exemplary aspects and embodiments described above, further aspects and embodiments will be further expanded upon in the figures and the following detailed descriptions.

### BRIEF DESCRIPTION OF THE FIGURES

The features, nature and advantages of the present disclosure will become more apparent from the detailed description set forth below when taken in conjunction with the drawings in which like reference characters identify correspondingly throughout. Identical structures elements or parts that appear in more than one figure are generally labeled with the same number in all the figures in which they appear. Alternatively, elements or parts that appear in more than one figure may be labeled with different numbers in the different figures in which they appear. The dimensions of the components and features in the figures were chosen for convenience and clarity of presentation and are not necessarily shown to scale. The figures are listed below.
**FIG. 1A** schematically illustrates a microcatheter comprising an outer layer including a plurality of sections, the plurality of sections made of different polymeric materials, according to some embodiments;
**FIG. 1B** schematically illustrates a perspective, cutaway view of the distal end of the microcatheter of **FIG. 1A** illustrating the outer layer, the strike layer, the inner layer, the braided skeleton located between the inner layer and the outer layer.
**FIG. 2A** schematically illustrates a 3.0 Fr embolization microcatheter with a fluid barrier forming section, according to some embodiments.
**FIG. 2B** schematically illustrates a magnified and partially exposed view of the distal end of the microcatheter of **FIG. 2A****,** according to some embodiments.
**FIG. 2C** schematically illustrates a magnified and partially exposed view of the distal tip of the microcatheter of **FIG. 2A****,** according to some embodiments.
**FIG. 2D** schematically illustrates a slit formed by selective cutting through the wall of a fluid barrier forming section, such as the fluid barrier forming section of the embolization microcatheter of **FIG. 2A****,** according to some embodiments.
**FIG. 3** schematically illustrates an optional slit pattern of the microcatheter of **FIG. 2A****.**
**FIG. 4** schematically illustrates another optional slit pattern of the microcatheter of **FIG. 2A****.**

### DETAILED DESCRIPTION OF THE INVENTION

The detailed description set forth below, in connection with the appended drawings, is intended as a description of various configurations and is not intended to represent the only configurations in which the concepts described herein may be practiced. The detailed description includes specific details for the purpose of providing a thorough understanding of the various concepts. However, it will also be apparent to one skilled in the art that these concepts may be practiced without specific details being presented herein. In some instances, well-known features may be omitted or simplified in order to avoid obscuring the disclosure.

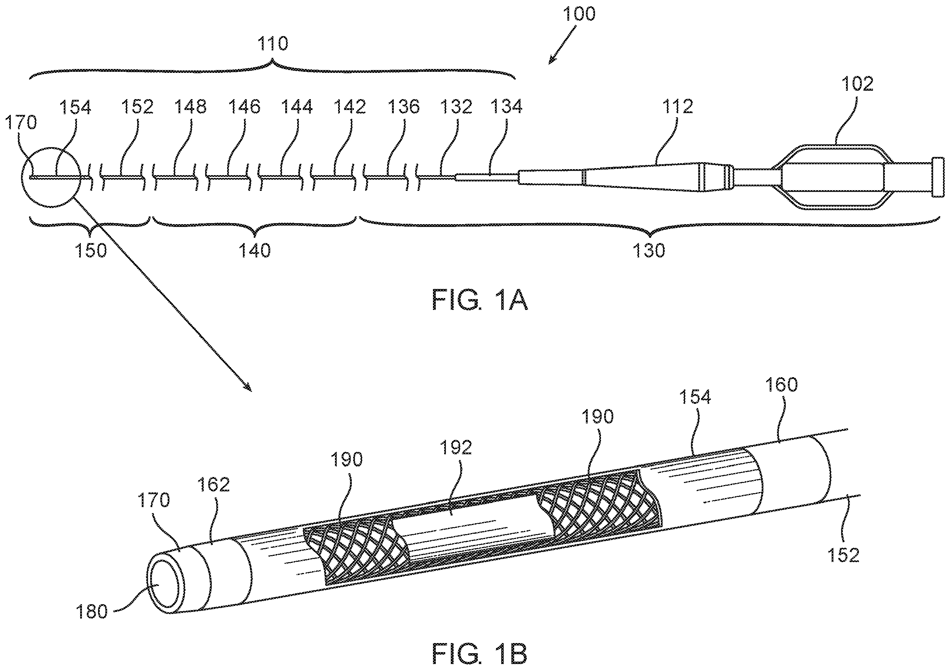

Reference is now made to **FIG. 1A****,** which schematically illustrates an embolization microcatheter **100,** and to **FIG. 1B** which illustrates magnified/exposed views of a distal part of **FIG. 1A****.**

As used herein, the terms "embolization", "transcatheter embolization", "transcatheter arterial embolization" and "TAE" may be used interchangeably and refer to the passage and lodging of an embolus within the bloodstream for therapeutic purposes, for example, as a hemostatic treatment of bleeding or as a treatment for some types of cancer by deliberately blocking blood vessels to starve the tumor cells.

Embolization microcatheter **100** is a 3.0 Fr microcatheter having an elongated tubular member **110** with an outer diameter of about 1.5 mm, or an outer diameter in the range of 1.0-1.5 mm, or 1.2-1.6 mm and an inner diameter in the range of 700-850 microns.

The proximal end **130** of microcatheter **100** includes a hub **102** which is molded on or otherwise attached to elongated tubular member **110** of microcatheter **100.**

Hub **102** is configured to allow access to the lumen of elongated tubular member **110** for a variety of functions, such as the injection of fluids or drugs, or the introduction of guidewires. Hub **102** includes a strain relief **112,** preferably mechanically coupled to hub **102.** Strain relief **112** may be made of a polymeric material and may, as illustrated, be tapered at its distal end. Strain relief **112** can be configured to provide structural support to elongated tubular member **110,** to prevent it from kinking.

According to some embodiments, the wall of elongated tubular member **110** may include a plurality of sections, each section characterized by the polymers utilized. According to some embodiments, a plurality of sections may include 3, 4, 5, 6, 7, 8, 9, 20 or more sections. Each possibility is a separate embodiment.

According to some embodiments, the different polymeric layers may contribute to different characteristics of the layer/section and thus of elongated tubular member **110.** For example, the different polymeric layers may contribute to the elasticity, flexibility, stretch-ability, strength, hardness, rigidity, ultimate tensile strength, elongation or any other characteristic of the layer and thus the microcatheter. Each possibility is a separate embodiment.

The proximal end **130** of elongated tubular member **110,** attached to strain relief **112,** includes an outer layer **132** made of a polyether block amide having a hardness of about 70D shore and/or a flexural modulus of about 74,000 psi (510 mpa). According to some embodiments, proximal end **132** may have a length of 600-1200mm (e.g. about 1000 mm).

Optionally, part of outer layer **132** may include a heat shrink material **134** covering the joint between strain relief **112** and elongated tubular member **110.**

Adjacent section **132,** is a second section **136** of elongated tubular member **110,** made of a polyether block amide having a hardness of about 60D-65D shore and/or a flexural modulus of about 41,000 psi (283 mpa). Section **136** may have a length of 20-60 mm, e.g. 40 mm.

Intermediate part **140** of elongated tubular member **110** includes section **142** having an outer layer which may be made of a polyether block amide or other suitable polymer having a hardness of about 55D shore and/or a flexural modulus of about 25,000 psi (172 mpa), section **144** having an outer layer made of a polymeric material having a hardness of about 40D shore such as a polycarbonate-based thermoplastic urethane having a hardness of about 40D shore, and/or a flexural modulus of about 10,000 psi (69 mpa) - 12,000 psi (83 mpa), section 146 having an outer layer made of a one or more polycarbonate-based thermoplastic urethanes having a hardness of about 95A shore, and section **148** having an outer layer made of a one or more polycarbonate-based thermoplastic urethanes having a hardness of about 85A shore.

Section **142** may have a length of about 30 to about 80 mm, e.g. 40 mm. Section **144,** may have a length of about 30 mm to about 70 mm (e.g. about 50 mm). Section **146** may have a length of about 80 mm to about 150 mm (e.g. about 130 mm). Section **148** may have a length of about 20 mm to about 60 mm (e.g. about 45 mm).

According to some embodiments, the distal end may refer to the distal 100mm, 50mm, 30mm, 20mm, 15mm, 10mm, 5mm or 2mm of microcatheter **100.** Each possibility is a separate embodiment. According to some embodiments, the distal end may refer to the part of the microcatheter extending between the proximal marker and distal end opening **180.**

According to some embodiments, the polymeric material of all sections in the intermediate part **140** is softer than that of the polymeric material of the wall section in proximal end **130** of tubular member **110.**

According to some embodiments, the polymeric material of section **148** is softer than that of section **146.** According to some embodiments, the polymeric material of section **146** is softer than that of section **144.** According to some embodiments, the polymeric material of section **144** is softer than that of section **142.**

Distal end **150** of elongated tubular member **110** includes sections **152** having an outer layer made of a polymeric material having a hardness of about 85A shore such as a polycarbonate-based thermoplastic urethane having a hardness of about 85A shore and section. The polymer of section **152** may further include a polymeric radiopaque marker, such as but not limited to tantalum powder; section **154** having an outer layer made of a polymeric material having a hardness of about 95A shore such as a polycarbonate-based thermoplastic urethane having a hardness of about 95A shore. According to some embodiments, the distal tip **170** of elongated tubular member **110** terminates with a distal end opening. According to some embodiments, the distal tip refers to the distal marker **162** and distal end opening **180.**

As used herein, the term "distal end opening" refers to the end opening of the microcatheter leading into the lumen thereof. According to some embodiments, distal end opening **180** defines the termination of the microcatheter at the distal end thereof. According to some embodiments, distal end opening **180** may have an inner diameter essentially equal to the inner diameter of the microcatheter lumen. According to some embodiments, the distal end opening **180** may have an inner diameter which is smaller than the inner diameter of the microcatheter lumen leading to a narrowing of the lumen toward the end thereof.

Section **152** includes a proximal marker **160** (seen in **FIG. 1B****),** section **154** terminates with a distal marker **162** (also seen in **FIG. 1B****).** According to some embodiments, distal end **150** may have a length of 10-20 mm. According to some embodiments, section **152** may have a length of 2-10 mm (e.g. about 6 mm). According to some embodiments, section **154** may have a length of 5-20 mm (e.g. about 10 mm). According to some embodiments, proximal marker **160** may be a radiopaque powder embedded in part of outer layer of section **152,** as essentially described herein. According to some embodiments, proximal marker **160** may be positioned approximately 5-20 mm, or 10-15 mm from the distal end opening **180.** According to some embodiments, distal marker **162** may be a radiopaque alloy submerged in outer layer **154.** According to some embodiments, distal marker **162** may be positioned approximately 1 mm proximally from distal end opening **180.**

Reference is now made to **FIG. 1B** which schematically illustrates a perspective, cutaway view of the distal part of distal end **150** of microcatheter **100** shown in **FIG. 1A** extending from proximal marker **160** to a tip **170** and encompassing section **154** of elongated tubular member **110.** As stated above, the wall of section **154.** As seen from the exploded view, underneath the outer layers is a braid **190.**

According to some embodiments, braid **190** extends along the entire length of tubular member **110.** Alternatively, braid **190** extends along only a portion of elongated tubular member **110,** such as only along section **154,** along sections **152** and **154,** along sections **152, 154** and **148,** along sections **152, 154, 148** and **146,** along sections **152, 154, 148, 146** and **144,** or along sections **152, 154, 148, 146, 144** and **142.** Each possibility is a separate embodiment.

According to some embodiments, braid **190** has a picks-per-inch (PPI) value ensuring that, in combination with a low durometer polymer, a flexible distal end is obtained, and in combination with a polymer having a higher durometer a relatively stiff proximal end is provided. According to some embodiments, the braided skeleton may have a wire arrangement of 75-150 Picks Per Inch (PPI) (29,53-59,06 picks per cm), 100-150 PPI (39,37-59,06 picks per cm) or 100-150 PPI (39,37-59,06 picks per cm). Each possibility is a separate embodiment. As a non-limiting example, the braided skeleton may have a wire arrangement of about 140 PPI (55,12 picks per cm) or about 145 PPI (57,09 picks per cm). Those skilled in the art will appreciate the term picks-per-inch (PPI) is a measurement of braid wire density and represents the number of picks (e.g. weft wires) per inch of braid.

As used herein the terms "braid" and "braided skeleton" may refer to a structural element, such as a tubal element formed of a plurality of interlaced wires. According to some embodiments, the braid may be formed of at least three interlaced wires forming a tube. According to some embodiments, the braid may include 8-48 wires or 12-32 wires. As a non-limiting example, the braid may include 16 wires. Each possibility is a separate element. According to some embodiments, the wires forming the braid may have a diameter in the range of 10-60 microns such as 15-40 microns or 20-30 microns or any other suitable diameter within the range of 10-60 microns. Each possibility is a separate embodiment. As a non-limiting example, the wires forming the braid may have a diameter of 25 microns. According to some embodiments, the skeleton may extend along essentially the entire length of the catheter. According to some embodiments, the braid may be made from tungsten, stainless steel, Nickel titanium (also referred to as Nitinol), nitinol, cobalt chrome, platinum iridium, nylon or any combination thereof. Each possibility is a separate embodiment.

According to some embodiments, at least some of the wires forming the braided skeleton may be braided in a same or opposite direction, i.e. left/right handed. Advantageously, the braiding structure allows good torque-ability (better than a coiled skeleton), low flexural rigidity (i.e. good flexibility), good push-ability (better than a coiled skeleton), and superior kink-resistance.

According to some embodiments, at least some of the wires forming the braided skeleton may be non-circular/round.

Underneath braid **190** is an inner liner **192,** which may be made of Polytetrafluoroethylene (PTFE). According to some embodiments, inner liner **192** may have a thickness of 10-30 micron or 10-25 micron. Each possibility is a separate embodiment. According to some embodiments, the liner is a film cast liner.

Advantageously, in order to compensate for the bulkiness distal marker **162,** the part of elongated tubular member **110** extending from the distal end of the distal marker **162,** to distal end opening **180** is devoid of inner liner **192,** thereby essentially maintaining the inner and outer diameters of elongated tubular member **110,** despite the bulkiness of distal marker **162,** which enables smooth delivery through a 5.0 Fr delivery catheter (not shown).

According to some embodiments, the total thickness of the wall of the distal end of the elongated tubular member does not exceed about 130 microns, at least along the part of the elongated tubular member to be inserted through the 5.0 Fr delivery catheter. According to some embodiments, the total thickness of the wall of the distal end of the elongated tubular member does not exceed about120 microns, at least along the part of the elongated tubular member to be inserted through the 5.0 Fr delivery catheter.

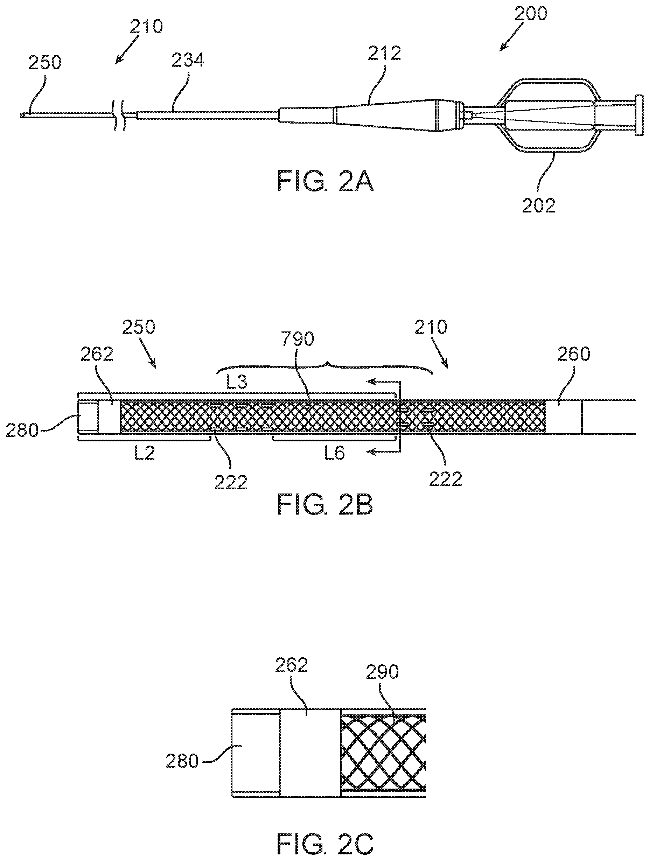

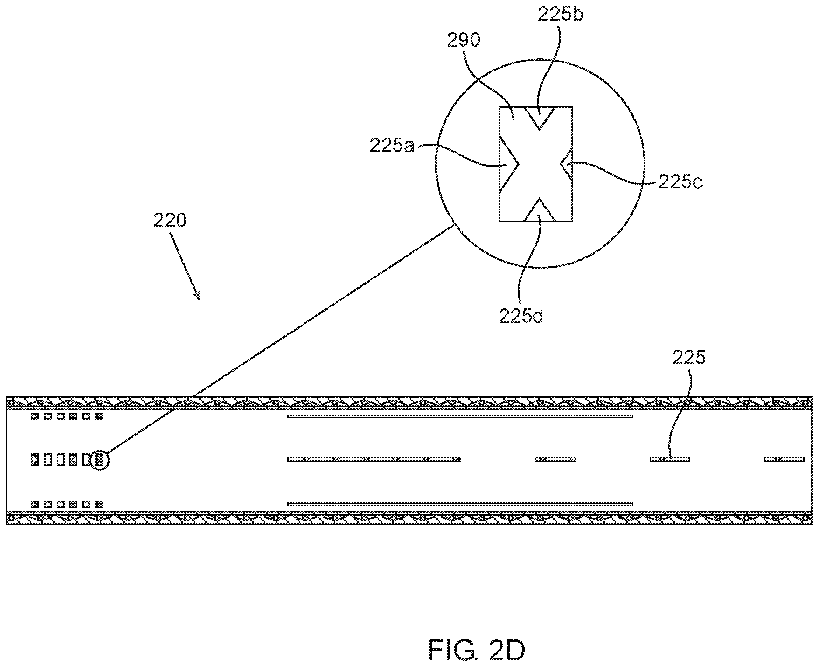

Reference is now made to **FIG. 2A****-****FIG. 2C****,** which schematically illustrate an embolization microcatheter **200** and magnified/exposed views of parts thereof. Embolization microcatheter **200** may be similar to embolization microcatheter **100** apart from embolization microcatheter **200** also including a filter **220.** Reference is also made to **FIG. 2D****,** which schematically illustrates the structure of filter **220.** Embolization microcatheter **200** is a 3.0 Fr microcatheter having an elongated tubular member **210** with an outer diameter in the range of about 1.0-1.5 mm, or in the range of about 1.2 mm - 1.6 mm and an inner diameter in the range of 700-850 microns. Embolization microcatheter **200** is particularly suitable for delivery of large embolization beads, such as but not limited to embolization beads having an average particle size of 500 microns or more, 600 microns or more, 700 microns or more, 800 microns or more, 900 microns or more and 1000 microns or more. Each possibility is a separate embodiment.

Filter **220** including a plurality of penetrating side openings formed in the wall of elongated tubular member **210,** is schematically illustrated in **FIG. 2B****.**

As used herein, the term "plurality" with reference to the side openings refers to 2 or more, 3 or more, 5 or more, 10 or more, 15 or more, 20 or more or 25 or more axial slits. Each possibility is a separate embodiment.

According to some embodiments, the filter **220** may be an integral part of elongated tubular member **110** and may extend along a length of 0.3mm-20mm, such as 1mm-10mm, 1mm-5mm, 1.5mm-5 mm, 2mm-5mm or any other in-between suitable length. Each possibility is a separate embodiment.

According to some embodiments, filter **220** may have a total open area, formed by the side openings, in the range of 0.2-1mm², 0.2-0.6mm², 0.3-1mm2, 0.3-0.5mm², 0.4-0.6mm², 0.5-1.5mm², 1.0-3.5mm², 1.5-4mm², 2.0-3.5mm² or any other suitable area within the range of 0.1-4mm². Each possibility is a separate embodiment. According to some embodiments, at least 5%, at least 10%, or at least 15% of filter **220** is open area formed by the side openings. According to some embodiments, 5%-30%, at least 7%-25%, 7%-20%, 5%-15% of filter **220** is open area formed by the side openings. Each possibility is a separate embodiment.

According to some embodiments, the side openings may be formed by selective cutting (e.g. selective laser cutting), that is, without cutting the wires forming braid **290** as illustrated in **FIG. 2D****.** According to some embodiments, the part of the liner positioned below the wires remains intact. According to some embodiments, both the polymeric layer and the inner liner positioned between the wires of braid **290** are penetrated when forming the slits. Advantageously, the selective cutting of the polymeric layer (leaving braid **290** essentially intact may provide subdivision of at least some of the side-openings into two or more sub-side-openings (here sub-side-opening **225a-225d)** separated by the braid but not by the polymeric outer layer.

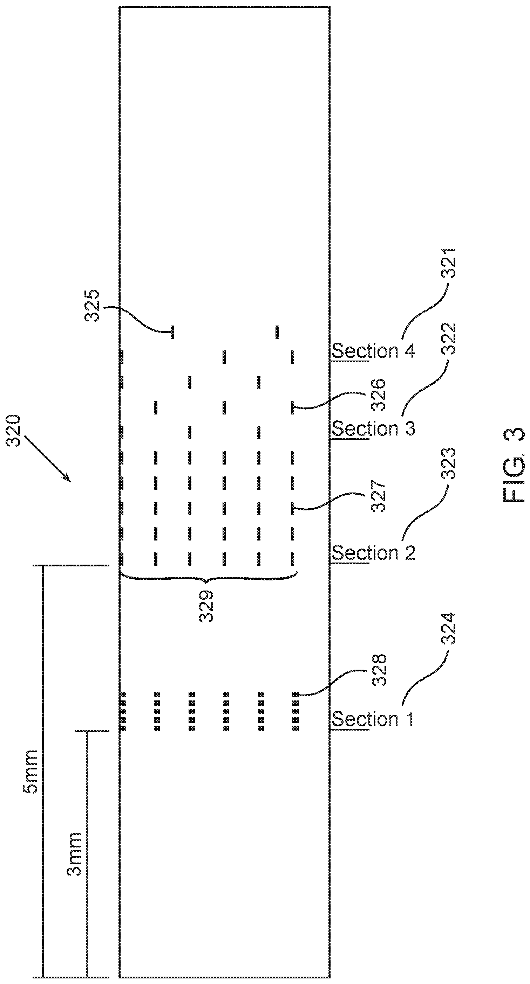

One optional structure of filter **220** is provided in **FIG. 3****.** As seen in **FIG. 3****,** filter **320** may include four filter sections **321, 322, 323** and **324** each filter section comprising a plurality of side openings **325, 326, 327** and **328,** distributed in circumferential rings around filter member **320.**

According to some embodiments, the proximal most filter section, filter section **321** has fewer side openings than filter sections distal thereto. According to some embodiments, filter section **321** may include 1-4 or 1-3 rings of side openings, such as, but not limited to 2 rings of side opening. According to some embodiments, each of the rings may include 1-5 side openings or 1-3 side openings, such as, but not limited to 2 side openings per ring. According to some embodiments, filter section **321** may include a total of 1-5, 2-4 side openings, such as but not limited to 2 side openings.

According to some embodiments, side openings of the proximal most circumferential sections, are circumferentially shifted relative to side openings in its neighboring circumferential section.

According to some embodiments, side openings **325** in a first ring of filter section **321** may be circumferentially shifted relative to side openings in its neighboring ring and/or circumferentially shifted relative to side openings **326** of filter section **322.**

According to some embodiments, filter section **322** may include 1-5 or 2-4 rings of side openings, such as, but not limited to 3 rings of side opening. According to some embodiments, each of the rings may include 1-5 side openings or 2-4 side openings, such as, but not limited to 3 side openings per ring. According to some embodiments, filter section **322** may include a total of 5-15, 6-10 side openings, such as but not limited to 9 side openings.

According to some embodiments, side openings **326** in a first ring of filter section **322** may be circumferentially shifted relative to side openings in its neighboring ring.

According to some embodiments, filter section **323** may include 2-10 or 3-7 rings of side openings, such as, but not limited to 5 rings of side opening. According to some embodiments, each of the rings may include 2-15 side openings or 4-8 side openings, such as, but not limited to 6 side openings per ring. According to some embodiments, filter section **323** may include a total of 10-50, 20-40 side openings, such as but not limited to 30 side openings.

According to some embodiments, side openings **325, 326** and **327** of filter sections **321, 322** and **323** respectively may be essentially rectangularly shaped axial slits. Due to the shape of the filter **220,** side openings **325, 326** and **327** may be conical, i.e. have a larger cross-section at the outer surface than at the inner surface of filter **220.**

The sizes provided below relate to measures made at the inner surface.

According to some embodiments, side openings **325, 326** and **327** may have a dimension of about 150x20 microns.

According to some embodiments, each ring of side openings **325, 326** and **327** of filter sections **321, 322** and **323** may be spaced apart from its neighboring ring by 100-200 microns or by 120-180 microns, such as but not limited to 150 microns.

According to some embodiments, the distal most of filter section, filter section **324** is positioned about 2-5 mm proximally to the distal end opening. According to some embodiments, the distal most of filter section, filter section **324** is positioned about 3 mm proximally to the distal end opening.

According to some embodiments, the side openings **328** of filter section **324** are essentially square shaped. Advantageously, the square-shaped size of the side openings **328** enables outflow of fluid, while ensuring minimal interference to the flow of beads towards distal end opening, despite the beads already being concentrated due to the outflow of fluid through side openings **325, 326** and **327.** According to some embodiments, filter section **324** may include 3-10 or 4-6 rings of side openings, such as, but not limited to 5 rings of side opening. According to some embodiments, each of the rings may include 3-10 side openings or 4-8 side openings, such as, but not limited to 6 side openings per ring.

According to some embodiments, side openings **328** of filter section **324** may have a dimension of about 50x50 microns.

According to some embodiments, the distal most ring of side openings **328** may be spaced apart by 1-10 mm or 2-7 mm, such as but not limited to 2 mm from a distal most of the rings of side openings **327** in filter section **323.**

According to some embodiments, the distal most ring of side openings **328** may be spaced apart by 1-10 mm or 2-5 mm, such as but not limited to 3 mm from the distal end opening.

According to some embodiments, each ring of side openings **328** of filter section **324** may be spaced apart from its neighboring ring by 20-100 microns or by 30-60 microns, such as but not limited to 50 microns.

According to some embodiments, the slits may be positioned at a same or a different longitudinal position. Each possibility is a separate embodiment. According to some embodiments the distribution of the slits may be staggered, zig-zagged or any other suitable even or uneven distribution.

Advantageously, the filter **320** may be configured for kink-free bending despite the plurality of slits formed in the wall thereof. According to some embodiments, the flexibility of the filter **220** is determined by the number of side openings, their minimal cross-sectional dimension, their width, length spacing, geometry, distance from distal outlet etc., as essentially described herein, may enable kink-free bending thereof.

As used herein the term "kink-free bending" may refer to a bending of filter **320,** which does impede flow therethrough. According to some embodiments, filter **320** may be configured for kink-free bending at an angle of about 180 degrees. According to some embodiments, filter **320** may be configured for kink-free bending at a minimum bending radius in the range of about 0.5 to 1.5 mm, for example 0.5 to 1.2, 0.5 to 1 mm, or any radius in-between.

Advantageously, microcatheter **300** including filter **320** provides effective reflux prevention, which requires a relatively high density of side openings, while a small kink-free radius (e.g. in the range of 0.5 to 1.5 mm) and tensile strength of at least 5N is still ensured.

According to some embodiments, the microcatheter **300** may have a length of at least 50 cm, at least 60 cm, at least 75 cm, or at least 1m. Each possibility is a separate embodiment.

According to some embodiments, the outer wall of the microcatheter **300** may be non-tapered essentially along its/their entire length.

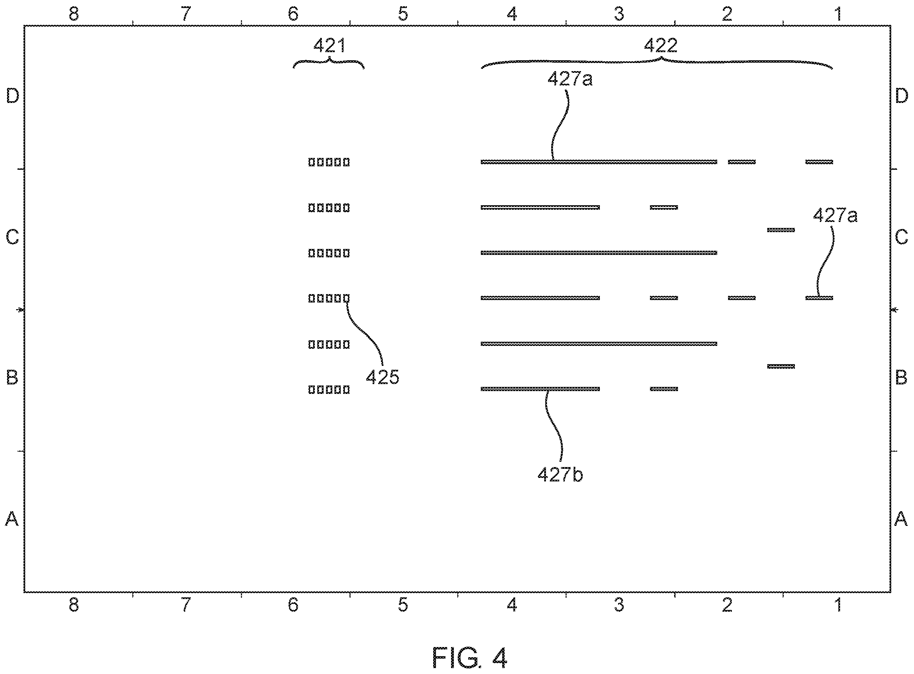

One optional structure of filter **220** is provided in **FIG. 4****.** As seen in **FIG. 4****,** filter **420** may include two filter sections **421** and **422** each filter section comprising a plurality of side openings **425** and **427,** distributed in circumferential rings around elongated tubular filter **420.**

Due to the shape of the filter **220,** side openings side openings **425** and **427** may be conical, i.e. have a larger cross-section at the outer surface than at the inner surface of filter **220.**

The sizes provided below relate to measures made at the inner surface.

According to some embodiments, filter section **421** may include 2-10 or 2-6 annular rings of side openings, such as, but not limited to 5 rings of side opening. According to some embodiments, each of the rings may include 1-10 side openings or 2-8 side openings, such as, but not limited to 6 side openings per ring. According to some embodiments, filter section **421** may include a total of 10-60 side openings or 20-40 side openings, such as but not limited to 30 side openings. According to some embodiments, side openings **425** may be essentially square or rectangularly shaped. According to some embodiments, side openings **425** may have a width of 50-150 microns or 50-100 microns such as e.g. about 80 microns. According to some embodiments, side openings **425** may have a length of 20-100 microns or such as e.g. about 50 microns. According to some embodiments, each of the rings of side opening may be spaced apart longitudinal from its neighboring ring by 20-100 microns, e.g. by approximately 50 microns. According to some embodiments, the distal most of side openings **425** may be positioned about 2-5 mm e.g. about 3 mm, from the distal end opening of the microcatheter.

Advantageously, the square-shaped size of the side openings **425** enables outflow of fluid while ensuring minimal interference to the flow of beads towards distal end opening, despite the beads already being concentrated due to the outflow of fluid through side openings **325, 326** and **327.**

According to some embodiments, filter section **422** may include 5-50 or 10-30 or 10-20 side opening **427.** According to some embodiments, the width of side openings **427** may by in the range of 10-50 or 10-30 microns, e.g. about 20 microns. According to some embodiments, at least some of side opening **427** may have a different length. According to some embodiments, at least some of side opening **427,** such as side opening **427a** may have a length in a range of about 1 mm - 4mm or about 2 mm - 3.5 mm, e.g. about 2.7 mm. According to some embodiments, at least some of side opening **427,** such as side opening **427b** may have a length in a range of about 0.8 mm - 2 mm or about 1 mm - 1.5 mm, e.g. about 1.35 mm. According to some embodiments, at least some of side opening **427,** such as side opening **427c** may have a length in a range of about 0.2 mm - 1 mm or about 0.2 mm - 0.5 mm, e.g. about 0.3 mm. Advantageously, filter **220** may be formed by selective cutting of the polymeric layer (leaving braid **290** essentially intact). According to some embodiments, at least some of side openings **427** may include sub-side-openings (illustrated as with numbers **225a-225d** in **FIG. 2D****)** separated by braid **290,** but not by the polymeric outer layer.

According to some embodiments, each of side openings **427** is spaced apart longitudinally from a neighboring side opening of side opening may be spaced apart from its neighboring side opening by 20-200 microns. According to some embodiments, the longitudinal spacing between side openings **427** may vary. According to some embodiments, at least some of side openings **427** are axially shifted vis-à-vis a neighboring side opening. According to some embodiments, the distal most of side openings **427** may be positioned about 3-10 mm e.g. about 5 mm, from the distal end opening of the microcatheter.

According to some embodiments, filter **420** may have a length of 5-15 mm or about 5-10 mm.

According to some embodiments, the slits may be positioned at a same or at a different longitudinal position. Each possibility is a separate embodiment. According to some embodiments the distribution of the slits may be staggered, zig-zagged or any other suitable even or uneven distribution.

Advantageously, the filter **420** may be configured for kink-free bending despite the plurality of slits formed in the wall thereof. According to some embodiments, the flexibility of the filter **220** is determined by the number of side openings, their minimal cross-sectional dimension, their width, length spacing, geometry, distance from distal outlet etc., as essentially described herein, may enable kink-free bending thereof.

As used herein the term "kink-free bending" may refer to a bending of filter **420,** which does impede flow therethrough. According to some embodiments, filter **420** may be configured for kink-free bending at an angle of about 180 degrees. According to some embodiments, filter **420** may be configured for kink-free bending at a minimum bending radius in the range of about 0.5 to 1.5 mm, for example 0.5 to 1.2, 0.5 to 1 mm, or any radius in-between.

Advantageously, microcatheter **400** including filter **420** provides effective reflux prevention, which requires a relatively high density of side openings, while a small kink-free radius (e.g. in the range of 0.5 to 1.5 mm) and tensile strength of at least 5N is still ensured.

According to some embodiments, the microcatheter **400** may have a length of at least 50 cm, at least 60 cm, at least 75 cm, or at least 1m. Each possibility is a separate embodiment. Each possibility is a separate embodiment.

According to some embodiments, the outer wall of the microcatheter **400** may be non-tapered essentially along its/their entire length.

As used herein, the terms "approximately" and "about" refer to +/-10%, or +/-5%, or +-2% vis-à-vis the range to which it refers. Each possibility is a separate embodiment.

While a number of exemplifying aspects and embodiments have been discussed above, those of skill in the art will envisage certain modifications, additions and sub-combinations thereof.

## Claims

1. An embolization microcatheter (100 or 200) comprising:
an elongated tubular member (110 or 210) forming a lumen, the elongated tubular member (110 or 210) terminating with a distal end opening (180), wherein a wall of the elongated tubular member (110 or 210) comprises a braid (190 or 290), a polymer formed around the braid and an inner liner (192) coating an inner surface of the wall; wherein the wall of the part of the elongated tubular member extending between a proximal radiopaque marker and the distal end opening (180) has a thickness of less than or equal to 130 microns; and
a distal radiopaque marker located in proximity to the distal end opening (180);
**characterized in that** the part of elongated tubular member (110 or 210) extending between the proximal end of the distal radiopaque marker and the distal end opening (180) is devoid of an inner liner.

2. The embolization microcatheter according to claim 1, wherein an outer diameter of the elongated tubular member (110 or 210) is less than or equal to 1.5 mm and an inner diameter of the elongated tubular member (110 or 210) is 700 microns or more; or wherein the thickness of the distal end (150) of the wall is less than or equal to 120 microns.

3. The embolization microcatheter according to any one of claims 1-2, further comprising a filter (220, 320, or 420) formed in the wall of the elongated tubular member (110 or 210), the filter (220, 320, or 420) comprising two or more filter segments (321, 322, 323, or 324), each filter segment comprising a plurality of side openings (325, 326, 327, or 328) penetrating the wall, the plurality of side openings (325, 326, 327, or 328) distributed circumferentially around the elongated tubular member (110 or 210).

4. The embolization microcatheter according to claim 3, wherein a proximal most of the filter segments (321) comprises fewer side openings than filter segments (322, 323, or 324) distal thereto.

5. The embolization microcatheter according to claim 3 or 4, wherein a distal most of the filter segments (324) is positioned 2-4 mm proximally to the distal end opening (180).

6. The embolization microcatheter according to any one of claims 3-5, wherein side openings (328) of the distal most filter segment (324) are essentially square shaped.

7. The embolization microcatheter according to claim 6, wherein the essentially square shaped side openings (328) have a dimension of 50x50 microns; or wherein the essentially square shaped side openings (328) have a dimension of 80x50 microns; or wherein the side openings (325, 326, or 327) of the remainder of the at least two filter segments (321, 322, or 323) are in a form of axial slits.

8. The embolization microcatheter according to claim 7, wherein the side openings (325, 326, or 327) of the remainder of the at least two filter segments (321, 322, or 323) have a width of 20 microns and wherein the length of the side openings (325, 326, or 327) of the remainder of the at least two filter segments (321, 322, or 323) varies between the side openings (325, 326, or 327); preferably wherein the side openings (325, 326, or 327) of the remainder of the at least two filter segments (321, 322, or 323) have a length of at least 200 microns.

9. The embolization microcatheter according to claim 8, wherein at least some of the side openings (325, 326, or 327)of the remainder of the at least two filter segments (321, 322, or 323) have dimension of 2700x20 microns; or wherein at least some of the side openings (325, 326, or 327) of the remainder of the at least two filter segments (321, 322, or 323) have a dimension of 1350x20 microns; or wherein at least some of the side openings (325, 326, or 327) of the remainder of the at least two filter segments (321, 322, or 323) have a dimension of 300x20 microns.

10. The embolization microcatheter according to any one of claims 1-9, wherein the braid (190 or 290) is made of tungsten.

11. The embolization microcatheter according to any one of claim 1-10, wherein the inner liner (192) comprises Polytetrafluoroethylene (PTFE).

12. The embolization microcatheter according to any one of claim 1-11, wherein the radiopaque marker comprises a metal marker band.

13. The embolization microcatheter according to any one of claims 1-12, wherein the radiopaque marker is located 0.5-2 mm proximally to the distal end opening (180).

14. The embolization microcatheter according to any one of claims 1-13, being suitable for delivery of embolization beads having a particle size of about 900 microns.

15. The embolization microcatheter according to any one of claims 1-14, having a length of at least 1 m.

## Patentansprüche

1. Embolisationsmikrokatheter (100 oder 200), umfassend:
ein längliches röhrenförmiges Element (110 oder 210), das ein Lumen bildet, wobei das längliche röhrenförmige Element (110 oder 210) mit einer distalen Endöffnung (180) endet, wobei eine Wand des länglichen röhrenförmigen Elements (110 oder 210) ein Geflecht (190 oder 290), ein um das Geflecht herum ausgebildetes Polymer und eine Innenauskleidung (192), die eine innere Oberfläche der Wand beschichtet, umfasst; wobei die Wand des sich zwischen einem proximalen röntgendichten Marker und der distalen Endöffnung (180) erstreckenden Teils des länglichen röhrenförmigen Elements eine Dicke von 130 Mikrometer oder weniger aufweist; und
einen in der Nähe der distalen Endöffnung (180) befindlichen distalen röntgendichten Marker;
**dadurch gekennzeichnet, dass** der sich zwischen dem proximalen Ende des distalen röntgendichten Markers und der distalen Endöffnung (180) erstreckende Teil des länglichen röhrenförmigen Elements (110 oder 210) frei von einer Innenauskleidung ist.

2. Embolisationsmikrokatheter nach Anspruch 1, wobei ein Außendurchmesser des länglichen röhrenförmigen Elements (110 oder 210) 1,5 mm oder weniger beträgt und ein Innendurchmesser des länglichen röhrenförmigen Elements (110 oder 210) 700 Mikrometer oder mehr beträgt; oder wobei die Dicke des distalen Endes (150) der Wand 120 Mikrometer oder weniger beträgt.

3. Embolisationsmikrokatheter nach einem der Ansprüche 1 bis 2, ferner umfassend einen in der Wand des länglichen röhrenförmigen Elements (110 oder 210) ausgebildeten Filter (220, 320 oder 420), wobei der Filter (220, 320 oder 420) zwei oder mehr Filtersegmente (321, 322, 323 oder 324) umfasst, wobei jedes Filtersegment mehrere Seitenöffnungen (325, 326, 327 oder 328) umfasst, die die Wand durchdringen, wobei die mehreren Seitenöffnungen (325, 326, 327, oder 328) in Umfangsrichtung um das längliche röhrenförmige Element (110 oder 210) herum verteilt sind.

4. Embolisationsmikrokatheter nach Anspruch 3, wobei ein am weitesten proximal angeordnetes Filtersegment (321) weniger Seitenöffnungen als distal dazu angeordnete Filtersegmente (322, 323 oder 324) umfasst.

5. Embolisationsmikrokatheter nach Anspruch 3 oder 4, wobei ein am weitesten distal angeordnetes Filtersegment (324) 2 bis 4 mm proximal zur distalen Endöffnung (180) positioniert ist.

6. Embolisationsmikrokatheter nach einem der Ansprüche 3 bis 5, wobei Seitenöffnungen (328) des am weitesten distal angeordneten Filtersegments (324) im Wesentlichen quadratisch geformt sind.

7. Embolisationsmikrokatheter nach Anspruch 6, wobei die im Wesentlichen quadratisch geformten Seitenöffnungen (328) eine Abmessung von 50 × 50 Mikrometern aufweisen oder wobei die im Wesentlichen quadratisch geformten Seitenöffnungen (328) eine Abmessung von 80 × 50 Mikrometern aufweisen oder wobei die Seitenöffnungen (325, 326 oder 327) des verbleibenden Teils der mindestens zwei Filtersegmente (321, 322 oder 323) in Form von axialen Schlitzen ausgebildet sind.

8. Embolisationsmikrokatheter nach Anspruch 7, wobei die Seitenöffnungen (325, 326 oder 327) des verbleibenden Teils der mindestens zwei Filtersegmente (321, 322 oder 323) eine Breite von 20 Mikrometern aufweisen und wobei die Länge der Seitenöffnungen (325, 326 oder 327) des verbleibenden Teils der mindestens zwei Filtersegmente (321, 322 oder 323) zwischen den Seitenöffnungen (325, 326 oder 327) variiert; wobei vorzugsweise die Seitenöffnungen (325, 326 oder 327) des verbleibenden Teils der mindestens zwei Filtersegmente (321, 322 oder 323) eine Länge von mindestens 200 Mikrometern aufweisen.

9. Embolisationsmikrokatheter nach Anspruch 8, wobei zumindest einige der Seitenöffnungen (325, 326 oder 327) des verbleibenden Teils der mindestens zwei Filtersegmente (321, 322 oder 323) eine Abmessung von 2.700 × 20 Mikrometern aufweisen oder wobei zumindest einige der Seitenöffnungen (325, 326 oder 327) des verbleibenden Teils der mindestens zwei Filtersegmente (321, 322 oder 323) eine Abmessung von 1.350 × 20 Mikrometern aufweisen oder wobei zumindest einige der Seitenöffnungen (325, 326 oder 327) des verbleibenden Teils der mindestens zwei Filtersegmente (321, 322 oder 323) eine Abmessung von 300 × 20 Mikrometern aufweisen.

10. Embolisationsmikrokatheter nach einem der Ansprüche 1 bis 9, wobei das Geflecht (190 oder 290) aus Wolfram hergestellt ist.

11. Embolisationsmikrokatheter nach einem der Ansprüche 1 bis 10, wobei die Innenauskleidung (192) Polytetrafluorethylen (PTFE) umfasst.

12. Embolisationsmikrokatheter nach einem der Ansprüche 1 bis 11, wobei der röntgendichte Marker ein Metallmarkerband umfasst.

13. Embolisationsmikrokatheter nach einem der Ansprüche 1 bis 12, wobei der röntgendichte Marker 0,5 bis 2 mm proximal zur distalen Endöffnung (180) angeordnet ist.

14. Embolisationsmikrokatheter nach einem der Ansprüche 1 bis 13, der zur Abgabe von Embolisationskügelchen mit einer Partikelgröße von etwa 900 Mikrometern geeignet ist.

15. Embolisationsmikrokatheter nach einem der Ansprüche 1 bis 14, der eine Länge von mindestens 1 m aufweist

## Revendications

1. Microcathéter d'embolisation (100 ou 200) comprenant :
un élément tubulaire allongé (110 ou 210) formant une lumière, l'élément tubulaire allongé (110 ou 210) se terminant par une ouverture d'extrémité distale (180), une paroi de l'élément tubulaire allongé (110 ou 210) comprenant une tresse (190 ou 290), un polymère formé autour de la tresse et un revêtement interne (192) recouvrant une surface interne de la paroi ; la paroi de la partie de l'élément tubulaire allongé qui s'étend entre un marqueur radio-opaque proximal et l'ouverture d'extrémité distale (180) ayant une épaisseur inférieure ou égale à 130 microns ; et
un marqueur radio-opaque distal situé à proximité de l'ouverture d'extrémité distale (180) ;
**caractérisé en ce que** la partie d'organe tubulaire allongé (110 ou 210) s'étendant entre l'extrémité proximale du marqueur radio-opaque distal et l'ouverture d'extrémité distale (180) est dépourvue de revêtement interne.

2. Microcathéter d'embolisation selon la revendication 1, un diamètre extérieur de l'élément tubulaire allongé (110 ou 210) étant inférieur ou égal à 1,5 mm et un diamètre intérieur de l'élément tubulaire allongé (110 ou 210) étant de 700 microns ou plus ; ou l'épaisseur de l'extrémité distale (150) de la paroi étant inférieure ou égale à 120 microns.

3. Microcathéter d'embolisation selon l'une quelconque des revendications 1 et 2, comprenant en outre un filtre (220, 320 ou 420) formé dans la paroi de l'élément tubulaire allongé (110 ou 210), le filtre (220, 320 ou 420) comprenant deux segments de filtre ou plus (321, 322, 323 ou 324), chaque segment de filtre comprenant une pluralité d'ouvertures latérales (325, 326, 327 ou 328) pénétrant dans la paroi, la pluralité d'ouvertures latérales (325, 326, 327, ou 328) étant répartis circonférentiellement autour de l'élément tubulaire allongé (110 ou 210).

4. Microcathéter d'embolisation selon la revendication 3, une plus grande partie proximale des segments de filtre (321) comprenant moins d'ouvertures latérales que des segments de filtre (322, 323 ou 324) distants de ceux-ci.

5. Microcathéter d'embolisation selon la revendication 3 ou 4, une plus grande partie distale des segments de filtre (324) étant positionnée à 2-4 mm de manière proximale par rapport à l'ouverture d'extrémité distale (180).

6. Microcathéter d'embolisation selon l'une quelconque des revendications 3 à 5, les ouvertures latérales (328) du segment filtrant le plus distal (324) étant essentiellement de forme carrée.

7. Microcathéter d'embolisation selon la revendication 6, les ouvertures latérales de forme essentiellement carrée (328) ayant une dimension de 50x50 microns ; ou les ouvertures latérales de forme essentiellement carrée (328) ayant une dimension de 80x50 microns ; ou les ouvertures latérales (325, 326 ou 327) du reste des au moins deux segments de filtre (321, 322 ou 323) ayant la forme de fentes axiales.

8. Microcathéter d'embolisation selon la revendication 7, les ouvertures latérales (325, 326 ou 327) du reste des au moins deux segments de filtre (321, 322 ou 323) ayant une largeur de 20 microns et la longueur des ouvertures latérales (325, 326 ou 327) du reste des au moins deux segments de filtre (321, 322 ou 323) variant entre les ouvertures latérales (325, 326 ou 327) ; de préférence, les ouvertures latérales (325, 326 ou 327) du reste des au moins deux segments de filtre (321, 322 ou 323) présentant une longueur d'au moins 200 microns.

9. Microcathéter d'embolisation selon la revendication 8, au moins certaines des ouvertures latérales (325, 326 ou 327) du reste des au moins deux segments de filtre (321, 322 ou 323) ayant une dimension de 2700x20 microns ; ou au moins certaines des ouvertures latérales (325, 326 ou 327) du reste des au moins deux segments de filtre (321, 322 ou 323) ayant une dimension de 1350x20 microns ; ou au moins certaines des ouvertures latérales (325, 326 ou 327) du reste des au moins deux segments de filtre (321, 322 ou 323) ayant une dimension de 300x20 microns.

10. Microcathéter d'embolisation selon l'une quelconque des revendications 1 à 9, la tresse (190 ou 290) étant constituée de tungstène.

11. Microcathéter d'embolisation selon l'une quelconque des revendications 1 à 10, le revêtement interne (192) comprenant du polytétrafluoroéthylène (PTFE).

12. Microcathéter d'embolisation selon l'une quelconque des revendications 1 à 11, le marqueur radio-opaque comprenant une bande de marqueur métallique.

13. Microcathéter d'embolisation selon l'une quelconque des revendications 1 à 12, le marqueur radio-opaque étant situé à 0.5-2 mm de manière proximale par rapport à l'ouverture d'extrémité distale (180).

14. Microcathéter d'embolisation selon l'une quelconque des revendications 1 à 13, convenant à la délivrance de billes d'embolisation ayant une taille de particule d'environ 900 microns.

15. Microcathéter d'embolisation selon l'une quelconque des revendications 1 à 14, ayant une longueur d'au moins 1 M.
